# EUROPEAN PATENT SPECIFICATION

(11) **EP 1 658 257 B1**
(45) Date of publication and mention of the grant of the patent: **18.11.2009**
(21) Application number: 04740513.9
(22) Date of filing: 01.07.2004
(51) Int. Cl.: C07C 41/03, C07C 43/02, C08G 65/28

(54) **PROCESS FOR THE ALKOXYLATION OF MONOOLS IN THE PRESENCE OF METALLO-ORGANIC FRAMEWORK MATERIALS**
VERFAHREN ZUR ALKOXYLIERUNG VON MONOOLEN IN GEGENWART VON ORGANOMETALLISCHEN GERÜSTMATERIALIEN
PROCEDE POUR L'ALCOXYLATION DE MONOALCOOLS EN PRESENCE DE MATERIAUX À STRUCTURE ORGANOMÉTALLIQUE

(30) Priority: 03.07.2003 US 611863
(43) Date of publication of application: 24.05.2006
(73) Proprietor: BASF SE, 67056 Ludwigshafen (DE); THE REGENTS OF THE UNIVERSITY OF MICHIGAN, Ann Arbor, Mi 48109-1280 (US)
(72) Inventor: MÜLLER, Ulrich, 67435 Neustadt (DE); Schubert, Olga, 67063 Ludwigshafen (DE); HESSE, Michael, 67549 Worms (DE); STÖSSER, Michael, 67141 Neuhofen (DE); HAAS, Peter, 67117 Limburgerhof (DE); YAGHI, Omar, M., Ann Arbor, MI 48103 (US)
(74) Representative: Isenbruck, Günter
(86) International application number: PCT/EP2004/007143
(87) International publication number: WO 2005/003069

(56) References cited:
- EP-A- 0 402 051
- US-A1- 2003 078 311
- US-B1- 6 624 318

## Description

The present invention relates to a process for the alkoxylation of monools in the presence of catalyst systems comprising a porous metallo-organic framework material of metal ions and a coordinately bound organic ligand which is at least bidentate. The invention further encompasses the use of polyoxyalkylene alcohols obtained in the process according to the present invention as tensides and flotation oils.

Polyoxyalkylene alcohols can be prepared e.g. by way of base or acid catalyzed polyaddition of alkaline oxides to polyfunctional organic compounds (starters). Suitable starters are e.g. water, alcohols, acids or amines or mixtures thereof which are selected according to the alcohol to be prepared. The drawback of the known preparation methods is that several elaborate purifying steps are necessary in order to separate the catalyst residue from the reaction product. Furthermore, the processes known in the art result in a mixture of various alkoxylation products, ranging from mono- to polyalkoxylated alcohols.

US-A 2003/078311 refers to a process for the alkoxylation or organic compounds using metal-organic framework materials to yield polyols.
EP-A 402 051 describes the ethoxylation of alcohols.

One object of the invention is to provide a process for the preparation of polyoxyalkylene alcohols from monools which does not show the drawbacks of the processes known in the art. In particular, the thus-obtained polyoxyalkylene alcohols should have a low impurity content, without requiring elaborate purifying steps of the starting materials and/or intermediate products. The process should furthermore not require elaborate purification steps in order to separate the catalyst from the reaction product(s). In particular, the process should give defined alkoxylation products with a defined alkoxylation range.

These objects are solved by a process for the alkoxylation of a monool with at least one alkoxylating agent to a polyoxyalkylene alcohol wherein the polyoxyalkylene alcohol comprises one to five alkoxy units and wherein a catalyst is employed which comprises a metallo-organic framework material of metal ions and at least bidentate coordinately bound organic ligands.

The present invention is drawn towards the alkoxylation of monools which are reacted with an alkoxylating agent, in general an alkylene oxide. Examples for monools which lend themselves for an alkoxylation according to the present invention are known to the person skilled in the art. Examples include monools of linear and branched alkyl groups having 1 to 30, preferably 1 to 20, in particular 1 to 15 carbon atoms, which alkyl groups may carry one or more aryl substituents, of homo- and polynuclear aromatic groups having 4 to 30, preferably 4 to 20, in particular 1 to 10 carbon atoms, which aromatic groups may carry one or more alkyl substituents, and of linear and branched alkenyl groups having 2 to 30, preferably 2 to 20, in particular 2 to 15 carbon atoms and which alkenyl groups may carry one or more aryl substituents. The alkyl, alkenyl and aryl groups may contain one or more hetero atoms in their carbon sceleton, and all said groups may carry one or more substituents other than those named. Examples for hetero atoms include N, O and S. Examples for substituents include halides and pseudohalides. Preferred alcohols should be liquid at room temperature.

Examples for preferred alcohols include Propylheptanol, Tridecanol H und Tridecanol N.

The alkoxylating agent is in general selected from epoxides having two to 30 carbon atoms and mixtures of two or more thereof. Preferably a linear or branched, cyclic or non-cyclic alkylene oxide having two to 24 C-atoms optionally carrying one or more substituents from the group consisting of aromatic groups, halides, hydroxyl groups, silyl groups, non-cyclic ether and ammonium groups is employed.

For the preferred group of alkylene oxides, the following are cited by way of example: ethylene oxide, 1,2-epoxypropane, 1,2-epoxy-2-methylpropane, 1,2-epoxybutane, 2,3-epoxybutane, 1,2-epoxy-3-methylbutane, 1,2-epoxypentane, 1,2-epoxy-3-methylpentane, 1,2-epoxyhexane, 1,2-epoxyheptane, 1,2-epoxyoctane, 1,2-epoxynonane, 1,2-epoxydecane, 1,2-epoxyundecane, 1,2-epoxydodecane, 1,2-epoxycyclopentane, 1,2-epoxycyclohexane, (2,3-epoxypropyl)benzene, vinyloxirane, 3-phenoxy-1,2-epoxypropane, 2,3-epoxymethyl ether, 2,3-epoxylethyl ether, 2,3-epoxyl isopropyl ether, 2,3-epoxyl-l-propanol, (3,4-epoxybutyl)stearate, 4,5-epoxypentylacetate, 2,3-epoxy propane methacrylate, 2,3-epoxy propane acrylat, glycidylbutyrate, methylglycidate, ethyl-2,3-epoxybutanoate, 4-(trimethylsilyl)butane-1,2-epoxide, 4-(triethylsilyl)butane-1,2-epoxide, 3-(perfluoromethyl)propane oxide, 3-(perfluoroethyl)propane oxide, 3-(perfluorobutyl)propane oxide, 4-(2,3-epoxypropyl)morpholine, 1-(oxirane-2-ylmethyl)pyrrolidin-2-one, styrene oxide, vinyl oxirane, aliphatic 1,2-alkylene oxides having 5 to 24 C-atoms, cyclopentane oxide, cyclohexane oxide, cyclododecatriane-(1,5,9)-monoxide and mixtures of two or more of the compounds cited.

Particularly preferred in the context of the present invention are ethylene oxide, propylene oxide, 1,2-epoxybutane, 2,3-epoxybutane, 1,2-epoxy-2-methylpropane, styrene oxide, vinyloxirane and any mixtures of two or more of the compounds cited. The most preferred epoxides are ethylene oxide, propylene oxide and mixtures of ethylene oxide with propylene oxide.

The process of preparing an epoxide by epoxidation is hereinafter described in detail by way of example, referring to propylene oxide.

Propylene oxide can be obtained by reacting propylene with oxygen; hydrogen and oxygen; hydrogen peroxide; organic hydroperoxides; or halohydrines, preferably by reacting propylene with hydrogen peroxide, more preferred by reacting propylene with hydrogen peroxide in the presence of a catalyst comprising a zeolithic material, particularly by reacting propylene with hydrogen peroxide in the presence of a catalyst comprising a titanium-containing zeolithic material having CS-1-structure.

It is particularly suitable to use hydrogen peroxide for the epoxidation.

The epoxidation is in principle known from e.g. DE 100 55 652.3 and further patent applications of the present applicant, such as DE 100 32 885.7, DE 100 32 884.9, DE 100 15 246.5, DE 199 36 547.4, DE 199 26 725.1, DE 198 47 629.9, DE 198 35 907.1, DE 197 23 950.1.

The alkoxylating agent obtained in the epoxidation step may be directly used without further treatment. It is, however, also possible within the present invention that the alkoxylating agent is treated beforehand, e.g. purified. As the purification method, mention can be made of a fine distillation. Suitable processes are e.g. disclosed in EP-B 0 557 116.

According to the present invention the alkoxylation reaction is carried out in the presence of a catalyst system which comprises a so called metallo-organic framework material.

Metal-organic framwork materials are known as such. They are described in, for example, US 5,648,508, EP-A-0 709 253, M. O'Keeffe et al., J. Sol. State Chm., 152 (2000) p. 3-20, H. Li et al., Nature 402 (1999) p. 276 seq., M. Eddaoudi et al., Topics in Catalysis 9 (1999) p. 105-111, B. Chen. Et al., Science 291 (2001) p. 1021-23. An inexpensive way for the preparation of said materials is disclosed in DE 101 11 230.0. The preparation of isoreticular MoF's is disclosed in WO 02/088148.

The metal-organic framework materials, as used in the present invention, comprise pores, particularly micro- and/or mesopores. Micropores are defined as pores having a diameter of 2 nm or below and mesopores as being pores having a diameter in the range of above 2 nm to 50 nm, respectively, according to the definition given in Pure Applied. Chem. 45, p. 71 seq., particularly on p. 79 (1976). The presence of the micro- and/or mesopores can be monitored by sorption measurements for determining the capacity of the metal-organic framework materials to take up nitrogen at 77 K according to DIN 66131 and/or DIN 66134. The specific surface areas cited in the context of the present invention are always determined according to DIN 66131 and/or DIN 66134.

For example, a type-I-form of the isothermal curve indicates the presence of micropores [see, for example, paragraph 4 of M. Eddaoudi et al., Topics in Catalysis 9 (1999)]. In a preferred embodiment, the specific surface area, as calculated according to the Langmuir model (DIN 66131, 66134) preferably is above 5 m²/g, further preferred above 10 m²/g, more preferably above 50 m²/g, particularly preferred above 500 m²/g and may increase to values of 3000 m²/g.

The metal ions forming the metal-organic framework material employed according to the present invention are preferably selected from the groups Ia, IIa, IIIa, IVa to VIIIa and Ib to VIb of the periodic system of the elements. Among these metals, particular reference is made to Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb, and Bi, more preferably Zn, Cu, Ni, Pd, Pt, Ru, Rh and Co. With respect to the metal ions of the aforementioned elements, particular reference is made to: Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, ^{V2+}, Nb³⁺, Ta³⁺, Cr³⁺, Mo³⁺, W³⁺, Mn³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir²⁺, Ir⁺, Ni²⁺, Ni⁺, Pd²⁺, Pd⁺, pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺ and Bi⁺.

With regard to the preferred metal ions and further details regarding the same, we particularly refer to: EP-A 0 790 253, particularly p. 10,1. 8-30, section "The Metal Ions".

In addition to the metal salts disclosed in EP-A 0 790 253 and US 5,648,508, other metallic compounds can be used, such as sulfates, phosphates and other compolex counter-ion metal salts of the main- and subgroup metals of the periodic system of the elements. Metal oxides, mixed oxides and mixutres of metal oxides and/or mixed oxides with or without a defined stoichiometry are preferred. All of the above mentioned metal compounds can be soluble or insoluble and they may be used as starting material either in form of a powder or as a shaped body or as any combination thereof.

The at least bidentate organic ligands present in the metall-organic framework material are capable of coordinating to the metal ion. Such ligands are known to the person skilled in the art. The at least bidentate organic ligand, is preferably selected from:
i) alkyl groups having from 1 to 10 carbon atoms,
ii) aryl groups having from 1 to 5 phenyl rings,
iii) alkyl and aryl amines carrying one or more alkyl groups having from 1 to 10 carbon atoms and/or one or more aryl groups having from 1 to 5 phenyl rings,
which are covalently substituted by at least one functional group X which can coordinately bind to the metal ion and which is selected from the group consisting of CO₂H, CS₂H, NO₂, SO₃H, Si(OH)₃, Ge(OH)₃, Sn(OH)₃, Si(SH)₄, Ge(SH)₄, Sn(SH)₃, PO₃H, AsO₃H, AsO₄Fi, P(SH)₃, As(SH)₃, CH(RSH)₂, C(RSH)₃, CH(RNH₂)₂, C(RNH₂)₃, CH(ROH)₂, C(ROH)₃, CH(RCN)₂, C(RCN)₃, wherein R is an alkyl group having from 1 to 5 carbon atoms, or an aryl group consisting of 1 to 2 phenyl rings, and CH(SH)₂, C(SH)₃, CH(NH₂)₂, C(NH₂)₂, CH(OH)₂, C(OH)₃, CH(CN)₂ and C(CN)₃. WO 02/088148 discloses bidentate organic ligand from the group of aromatic compounds which can carry one or more substituents. The content of WO 02/088148, pages 8 - 14 is herein fully incorporated by reference.

Particularly to be mentioned are substituted and unsubstituted aliphatic α,ω̅-dicarboxylic acids, substituted or unsubstituted, mono- or polynuclear aromatic di-, tri- and tetracarboxylic acids and substituted or unsubstituted, aromatic di-, tri- and tetracarboxylic acids, having one or more nuclei, and carying at least one hetero atom

Preferred ligands are selected from 1,3,5-benzene tricarboxylic acid (BCT), NDC (naphthalene dicarboxylate), BDC (benzene dicarboxylate), BTC (benzene tricarboxylate), BTB (benzene tribenzoate), and DHBC (2,5-dihydroxyterephtalic acid).

DHBC is the most preferred ligand. Besides the at least bidentate organic ligand, the framework material as used in accordance with the present invention may also comprise one or more monodentate ligands, which are preferably selected from the following monodentate substances and/or derivatives thereof:
a. alkyl amines and their corresponding alkyl ammonium salts, containing linear, branched, or cyclic aliphatic groups, having from 1 to 20 carbon atoms (and their corresponding ammonium salts);
b. aryl amines and their corresponding aryl ammonium salts having from 1 to 5 phenyl rings;
c. alkyl phosphonium salts, containing linear, branched, or cyclic aliphatic groups, having from 1 to 20 carbon atoms;
d. aryl phosphonium salts, having from 1 to 5 phenyl rings;
e. alkyl organic acids and the corresponding alkyl organic anions (and salts) containing linear, branched, or cyclic aliphatic groups, having from 1 to 20 carbon atoms;
f. aryl organic acids and their corresponding aryl organic anions and salts, having from 1 to 5 phenyl rings;
g. aliphatic alcohols, containing linear, branched, or cyclic aliphatic groups, having from 1 to 20 carbon atoms;
h. aryl alcohols having from 1 to 5 phenyl rings;
i. inorganic anions from the group consisting of:
   sulfate, nitrate, nitrite, sulfite, bisulfite, phosphate, hydrogen phosphate, dihydrogen phosphate, diphosphate, triphosphate, phosphate, phosphite, chloride, chlorate, bromide, bromate, iodide, iodate, carbonate, bicarbonate, and the corresponding acids and salts of the aforementioned inorganic anions,
j. ammonia, carbon dioxide, methane, oxygen, ethylene, hexane, benzene, toluene, xylene, chlorobenzene, nitrobenzene, naphthalene, thiophene, pyridine, acetone, 1-2-dichloroethane, methylenechloride, tetrahydrofuran, ehtanolamine, triethylamine and trifluoromethylsulfonic acid.

Further details regarding the at least bidentate organic ligand and the mono-dentate substances, from which the ligands of the framework material as used in the present application are derived, may be deduced from EP-A 0 790 253.

Within the present application, framework materials of the kind described herein, which comprise Zn2⁺ as a metal ion and ligands derived from terephthalic acid as the bidentate ligand, are particularly preferred.

Further metal ions, at least bidentate and monodentats organic ligands which are useful for the preparation of the framework materials used in the present invention as well as processes for their preparation are particularly disclosed in EP-A 0 790 253, US 5,648,508 and DE 10111230.0.

As solvents, which are particularly useful for the preparation of MOF-5, in addition to the solvents disclosed in the above-referenced literature dimethyl formamide, diethyl formamide and N-methylpyrrolidone, alone, in combination with each other or in combination with other solvents may be used. Within the preparation of the framework materials, particularly within the preparation of MOF-5, the solvents and mother liquors can be recycled after crystallization.

The pore sizes of the metal-organic framework can be adjusted by selecting suitable bidendate ligands (=linkers). Generally, the larger the linker, the larger the pore size. Any pore size that is still supported by a the metal-organic framework in the absence of a host and at temperatures of at least 200°C is conceivable. Pore sizes ranging from 0,2 nm to 30 nm are preferred, with pore sizes ranging from 0,3 nm to 3 nm being particularly preferred.

In the following, examples of metal-organic framework materials (MOFs) are given to illustrate the general concept given above.

By way of example, a list of metal-organic framework materials already synthesized and characterized is given below. This also includes novel isoreticular metal organic framework materials (IR-MOFs), which may be used in the context of the present application. Such materials having the same framework topology while displaying different pore sizes and crystal densities are described, for example in M. Eddouadi et al., Science 295 (2002) 469.

The solvents used are of particular importance for the synthesis of these materials and are therefore mentioned in the table. The values for the cell parameters (angles Δ, E and ϑ as well as the spacings a, b and c, given in Angstrom) have been obtained by x-ray diffraction and represent the space group given in the table as well.

| **MOF-n** | **Ingredients molar ratios M+L** | **Solvents** | α | β | γ | **a** | **b** | **c** | **Space Group** |
|---|---|---|---|---|---|---|---|---|---|
| MOF-0 | Zn(NO₃)₂·6H₂O | Ethanol | 90 | 90 | 120 | 16.711 | 16.711 | 14.189 | P6(3)/ |
| | H₃(BTC) | | | | | | | | Mcm |
| MOF-2 | Zn(NO₃)₂·6H₂O | DMF | 90 | 102.8 | 90 | 6.718 | 15.49 | 12.43 | P2(1)/n |
| | (0.246 mmol) | Toluene | | | | | | | |
| | H₂(BDC) | | | | | | | | |
| | 0.241 mmol) | | | | | | | | |
| MOF-3 | Zn(NO₃)₂·6H₂O | DMF | 99.72 | 111.11 | 108.4 | 9.726 | 9.911 | 10.45 | P-1 |
| | (1.89 mmol) | MeOH | | | | | | | |
| | H₂(BDC) | | | | | | | | |
| | (1.93mmol) | | | | | | | | |
| MOF-4 | Zn(NO₃)₂·6H₂O | Ethanol | 90 | 90 | 90 | 14.728 | 14.728 | 14.728 | P2(1)3 |
| | (1.00 mmol) | | | | | | | | |
| | H₃(BTC) | | | | | | | | |
| | (0.5 mmol) | | | | | | | | |
| MOF-5 | Zn(NO₃)₂·6H₂O | DMF | 90 | 90 | 90 | 25.669 | 25.669 | 25.669 | Fm-3m |
| | (2.22 mmol) | Chloroben- | | | | | | | |
| | H₂(BDC) | zene | | | | | | | |
| | (2.17 mmol) | | | | | | | | |
| MOF-38 | Zn(NO₃)₂·6H₂O | DMF | 90 | 90 | 90 | 20.657 | 20.657 | 17.84 | I4cm |
| | (0.27 mmol) | Chloroben | | | | | | | |
| | H₃(BTC) | zene | | | | | | | |
| | (0.15 mmol) | | | | | | | | |
| MOF-31 | Zn(NO₃)₂·6H₂O | Ethanol | 90 | 90 | 90 | 10.821 | 10.821 | 10.821 | Pn(-3)m |
| Zn(ADC)₂ | 0.4 mmol | | | | | | | | |
| | H₂(ADC) | | | | | | | | |
| | 0.8 mmol | | | | | | | | |
| MOF-12 | Zn(NO₃)₂·6H₂O | Ethanol | 90 | 90 | 90 | 15.745 | 16.907 | 18.167 | Pbca |
| Zn₂(ATC) | 0.3 mmol | | | | | | | | |
| | H₄(ATC) | | | | | | | | |
| | 0.15 mmol | | | | | | | | |
| MOF-20 | Zn(NO₃)₂·6H₂O | DMF | 90 | 92.13 | 90 | 8.13 | 16.444 | 12.807 | P2(1)/c |
| ZnNDC | 0.37 mmol | Chloroben- | | | | | | | |
| | H₂NDC | zene | | | | | | | |
| | 0.36 mmol | | | | | | | | |
| MOF-37 | Zn(NO₃)₂·6H₂O | DEF | 72.38 | 83.16 | 84.33 | 9.952 | 11.576 | 15.556 | P-1 |
| | 0.2 mmol | Chloro- | | | | | | | |
| | H₂NDC | Benzene | | | | | | | |
| | 0.2 mmol | | | | | | | | |
| MOF-8 | Tb(NO₃)₃·5H₂O | DMSO | 90 | 115.7 | 90 | 19.83 | 9.822 | 19.183 | C2/c |
| Tb₂(ADC) | 0.10 mmol | MeOH | | | | | | | |
| | H₂ADC | | | | | | | | |
| | 0.20 mmol | | | | | | | | |
| MOF-9 | Tb(NO₃)₃·5H₂O | DMSO | 90 | 102.09 | 90 | 27.056 | 16.795 | 28.139 | C2/c |
| Tb₂ (ADC) | 0.08 mmol | | | | | | | | |
| | H₂ADB | | | | | | | | |
| | 0.12 mmol | | | | | | | | |
| MOF-6 | Tb(NO₃)₃·5H₂O | DMF | 90 | 91.28 | 90 | 17.599 | 19.996 | 10.545 | P21/c |
| | 0.30 mmol | MeOH | | | | | | | |
| | H₂ (BDC) | | | | | | | | |
| | 0.30 mmol | | | | | | | | |
| MOF-7 | Tb(NO₃)₃·5H₂O | H₂O | 102.3 | 91.12 | 101.5 | 6.142 | 10.069 | 10.096 | P-1 |
| | 0.15 mmol H₂(BDC) 0.15 mmol | | | | | | | | |
| MOF-69A | Zn(NO₃)₂·6H₂O | DEF | 90 | 111.6 | 90 | 23.12 | 20.92 | 12 | C2/c |
| | 0.083 mmol | H₂O₂ | | | | | | | |
| | 4,4'BDC | MeNH₂ | | | | | | | |
| | 0.041 mmol | | | | | | | | |
| MOF-69B | Zn(NO₃)₂·6H₂O | DEF | 90 | 95.3 | 90 | 20.17 | 18.55 | 12.16 | C2/c |
| | 0.083 mmol | H₂O₂ | | | | | | | |
| | 2,6-NCD | MeNH₂ | | | | | | | |
| | 0.041 mmol | | | | | | | | |
| MOF-11 | Cu(NO₃)₂·2.5H₂O | H₂O | 90 | 93.86 | 90 | 12.987 | 11.22 | 11.336 | C2/c |
| Cu₂(ATC) | 0.47 mmol | | | | | | | | |
| | H₂ATC | | | | | | | | |
| | 0.22 mmol | | | | | | | | |
| MOF-11 | | | 90 | 90 | 90 | 8.4671 | 8.4671 | 14.44 | P42/ |
| Cu₂(ATC) dehydr. | | | | | | | | | mmc |
| MOF-14 | Cu(NO₃)₂·2.5H₂O | H₂O | 90 | 90 | 90 | 26.946 | 26.946 | 26.946 | Im-3 |
| Cu₃ (BTB) | 0.28 mmol | DMF | | | | | | | |
| | H₃BTB | EtOH | | | | | | | |
| | 0.052 mmol | | | | | | | | |
| MOF-32 | Cd(NO₃)₂·4H₂O | H₂O | 90 | 90 | 90 | 13.468 | 13.468 | 13.468 | P(-4)3m |
| Cd(ATC) | 0.24 mmol | NaOH | | | | | | | |
| | H₄ATC | | | | | | | | |
| | 0.10 mmol | | | | | | | | |
| MOF-33 | ZnCl₂ | H₂O | 90 | 90 | 90 | 19.561 | 15.255 | 23.404 | Imma |
| Zn₂ (ATB) | 0.15 mmol | DMF | | | | | | | |
| | H₄ATB | EtOH | | | | | | | |
| | 0.02 mmol | | | | | | | | |
| MOF-34 | Ni(NO₃)₂·6H₂O | H₂O | 90 | 90 | 90 | 10.066 | 11.163 | 19.201 | P2₁2₁2₁ |
| Ni(ATC) | 0.24 mmol | NaOH | | | | | | | |
| | H₄ATC | | | | | | | | |
| | 0.10 mmol | | | | | | | | |
| MOF-36 | Zn(NO₃)₂·4H₂O | H₂O | 90 | 90 | 90 | 15.745 | 16.907 | 18.167 | Pbca |
| Zn₂ (MTB) | 0.20 mmol | DMF | | | | | | | |
| | H₄MTB | | | | | | | | |
| | 0.04 mmol | | | | | | | | |
| MOF-39 | Zn(NO₃)₂ 4H₂O | H₂O | 90 | 90 | 90 | 17.158 | 21.591 | 25.308 | Pnma |
| Zn₃O(HBTB) | 0.27 mmol | DMF | | | | | | | |
| | H₃BTB | EtOH | | | | | | | |
| | 0.07 mmol | | | | | | | | |
| N0305 | FeCl₂·4H₂O | DMF | 90 | 90 | 120 | 8.2692 | 8.2692 | 63.566 | R-3c |
| | 5.03 mmol | | | | | | | | |
| | formic acid | | | | | | | | |
| | 86.90 mmol | | | | | | | | |
| NO306A | FeCl₂·4H₂O | DEF | 90 | 90 | 90 | 9.9364 | 18.374 | 18.374 | Pbcn |
| | 5.03 mmol | | | | | | | | |
| | formic acid | | | | | | | | |
| | 86.90 mmol | | | | | | | | |
| NO29 | Mn(Ac)₂·4H₂O | DMF | 120 | 90 | 90 | 14.16 | 33.521 | 33.521 | P-1 |
| MOF-0 like | 0.46 mmol | | | | | | | | |
| | H₃BTC | | | | | | | | |
| | 0.69 mmol | | | | | | | | |
| BPR48 | Zn(NO₃)₂ 6H₂O | DMSO | 90 | 90 | 90 | 14.5 | 17.04 | 18.02 | Pbca |
| A2 | 0.012 mmol | Toluene | | | | | | | |
| | H₂BDC | | | | | | | | |
| | 0.012 mmol | | | | | | | | |
| BPR69 | Cd(NO₃)₂ 4H₂O | DMSO | 90 | 98.76 | 90 | 14.16 | 15.72 | 17.66 | Cc |
| B1 | 0.0212 mmol | | | | | | | | |
| | H₂BDC | | | | | | | | |
| | 0.0428 mmol | | | | | | | | |
| BPR92 | Co(NO₃)₂·6H₂O | NMP | 106.3 | 107.63 | 107.2 | 7.5308 | 10.942 | 11.025 | P1 |
| A2 | 0.018 mmol | | | | | | | | |
| | H₂BDC | | | | | | | | |
| | 0.018 mmol | | | | | | | | |
| BPR95 | Cd(NO₃)₂ 4H₂O | NMP | 90 | 112.8 | 90 | 14.460 | 11.085 | 15.829 | P2(1)/n |
| C5 | 0.012 mmol | | | | | | | | |
| | H₂BDC | | | | | | | | |
| | 0.36 mmol | | | | | | | | |
| Cu C₆H₄O₆ | Cu(NO₃)₂·2.5H₂O | DMF | 90 | 105.29 | 90 | 15.259 | 14.816 | 14.13 | P2(1)/c |
| | 0.370 mmol | Chlorob | | | | | | | |
| | H₂BDC(OH)₂ | enzene | | | | | | | |
| | 0.37 mmol | | | | | | | | |
| M(BTC) | Co(SO₄) H₂O | DMF | Same as MOF-0 | | | | | | |
| MOF-0like | 0.055 mmol | | | | | | | | |
| | H₃BTC | | | | | | | | |
| | 0.037 mmol | | | | | | | | |
| Tb(C₆H₄O₆) | Tb(NO₃)₃·5H₂O | DMF | 104.6 | 107.9 | 97.147 | 10.491 | 10.981 | 12.541 | P-1 |
| | 0.370 mmol | chlorobe | | | | | | | |
| | H₂(C₆H₄O₆) | nzene | | | | | | | |
| | 0.56 mmol | | | | | | | | |
| Zn (C₂O₄) | ZnCl₂ | DMF | 90 | 120 | 90 | 9.4168 | 9.4168 | 8.464 | P(-3)1m |
| | 0.370 mmol | chlorobe- | | | | | | | |
| | oxalic acid | nzene | | | | | | | |
| | 0.37 mmol | | | | | | | | |
| Co(CHO) | Co(NO₃)₂·5H₂O | DMF | 90 | 91.32 | 90 | 11.328 | 10.049 | 14.854 | P2(1)/n |
| | 0.043 mmol | | | | | | | | |
| | formic acid | | | | | | | | |
| | 1.60 mmol | | | | | | | | |
| Cd(CHO) | Cd(NO₃)₂·4H₂O | DMF | 90 | 120 | 90 | 8.5168 | 8.5168 | 22.674 | R-3c |
| | 0.185 mmol | | | | | | | | |
| | formic acid | | | | | | | | |
| | 0.185 mmol | | | | | | | | |
| Cu(C₃H₂O₄) | Cu(NO₃)₂·2.5H₂O | DMF | 90 | 90 | 90 | 8.366 | 8.366 | 11.919 | P43 |
| | 0.043 mmol | | | | | | | | |
| | malonic acid | | | | | | | | |
| | 0.192 mmol | | | | | | | | |
| Zn₆ (NDC)₅ | Zn(NO₃)₂·6H₂O | DMF | 90 | 95.902 | 90 | 19.504 | 16.482 | 14.64 | C2/m |
| MOF-48 | 0.097 mmol | chlorobe | | | | | | | |
| | 14 NDC | nzene | | | | | | | |
| | 0.069 mmol | H₂O₂ | | | | | | | |
| MOF-47 | Zn(NO₃)₂ 6H₂O | DMF | 90 | 92.55 | 90 | 11.303 | 16.029 | 17.535 | P2(1)/c |
| | 0.185 mmol | Chlorob- | | | | | | | |
| | H₂(BDC[CH₃]₄) | enzene | | | | | | | |
| | 0.185 mmol | H₂O₂ | | | | | | | |
| MO25 | Cu(NO₃)₂·2.5H₂O | DMF | 90 | 112.0 | 90 | 23.880 | 16.834 | 18.389 | P2(1)/c |
| | 0.084 mmol | | | | | | | | |
| | BPhDC | | | | | | | | |
| | 0.085 mmol | | | | | | | | |
| Cu-Thio | Cu(NO₃)₂·2.5H₂O | DEF | 90 | 113.6 | 90 | 15.4747 | 14.514 | 14.032 | P2(1)/c |
| | 0.084 mmol | | | | | | | | |
| | thiophene | | | | | | | | |
| | dicarboxylic | | | | | | | | |
| | 0.085 mmol | | | | | | | | |
| ClBDC1 | Cu(NO₃)₂·2.5H₂O0.0 | DMF | 90 | 105.6 | 90 | 14.911 | 15.622 | 18.413 | C2/c |
| | 84 mmol | | | | | | | | |
| | H₂(BDCCl₂) | | | | | | | | |
| | 0.085 mmol | | | | | | | | |
| MOF-101 | Cu(NO₃)₂·2.5H₂O | DMF | 90 | 90 | 90 | 21.607 | 20.607 | 20.073 | Fm3m |
| | 0.084 mmol | | | | | | | | |
| | BrBDC | | | | | | | | |
| | 0.085 mmol | | | | | | | | |
| Zn₃(BTC)₂ | ZnCl₂ | DMF | 90 | 90 | 90 | 26.572 | 26.572 | 26.572 | Fm-3m |
| | 0.033 mmol | EtOH | | | | | | | |
| | H₃BTC | Base | | | | | | | |
| | 0.033 mmol | Added | | | | | | | |
| MOF-j | Co(CH₃CO₂)₂·4H₂O | H₂O | 90 | 112.0 | 90 | 17.482 | 12.963 | 6.559 | C2 |
| | (1.65 mmol) | | | | | | | | |
| | H₃(BZC) | | | | | | | | |
| | (0.95 mmol) | | | | | | | | |
| MOF-n | Zn(NO₃)₂·6H₂O | ethanol | 90 | 90 | 120 | 16.711 | 16.711 | 14.189 | P6(3)/mcm |
| | H₃ (BTC) | | | | | | | | |
| PbBDC | Pb(NO₃)₂ | DMF | 90 | 102.7 | 90 | 8.3639 | 17.991 | 9.9617 | P2(1)/n |
| | (0.181 mmol) | Ethanol | | | | | | | |
| | H₂(BDC) | | | | | | | | |
| | (0.181 mmol) | | | | | | | | |
| Znhex | Zn(NO₃)₂·6H₂O | DMF | 90 | 90 | 120 | 37.1165 | 37.117 | 30.019 | P3(1)c |
| | (0.171 mmol) | p-xylene | | | | | | | |
| | H₃BTB | ethanol | | | | | | | |
| | (0.114 mmol) | | | | | | | | |
| AS16 | FeBr₂ | DMF | 90 | 90.13 | 90 | 7.2595 | 8.7894 | 19.484 | P2(1)c |
| | 0.927 mmol | anhydr. | | | | | | | |
| | H₂(BDC) | | | | | | | | |
| | 0.927 mmol | | | | | | | | |
| AS27-2 | FeBr₂ | DMF | 90 | 90 | 90 | 26.735 | 26.735 | 26.735 | Fm3m |
| | 0.927 mmol | anhydr. | | | | | | | |
| | H₃(BDC) | | | | | | | | |
| | 0.464 mmol | | | | | | | | |
| AS32 | FeCl₃ | DMF | 90 | 90 | 120 | 12.535 | 12.535 | 18.479 | P6(2)c |
| | 1.23 mmol | anhydr. | | | | | | | |
| | H₂(BDC) | Ethanol | | | | | | | |
| | 1.23 mmol | | | | | | | | |
| AS54-3 | FeBr₂ | DMF | 90 | 109.98 | 90 | 12.019 | 15.286 | 14.399 | C2 |
| | 0.927 | anhydr. | | | | | | | |
| | BPDC | n- | | | | | | | |
| | 0.927 mmol | propanol | | | | | | | |
| AS61-4 | FeBr₂ | Pyridine | 90 | 90 | 120 | 13.017 | 13.017 | 14.896 | P6(2)c |
| | 0.927 mmol | anhydr. | | | | | | | |
| | m-BDC | | | | | | | | |
| | 0.927 mmol | | | | | | | | |
| AS68-7 | FeBr₂ | DMF | 90 | 90 | 90 | 18.3407 | 10.036 | 18.039 | Pca2₁ |
| | 0.927 mmol | anhydr. | | | | | | | |
| | m-BDC | Pyridine | | | | | | | |
| | 1.204 mmol | | | | | | | | |
| Zn(ADC) | Zn(NO₃)₂·6H₂O | DMF | 90 | 99.85 | 90 | 16.764 | 9.349 | 9.635 | C2/c |
| | 0.37 mmol | Chlorob | | | | | | | |
| | H₂(ADC) | enzene | | | | | | | |
| | 0.36 mmol | | | | | | | | |
| MOF-12 | Zn(NO₃)₂·6H₂O | Ethanol | 90 | 90 | 90 | 15.745 | 16.907 | 18.167 | Pbca |
| Zn₂ (ATC) | 0.30 mmol | | | | | | | | |
| | H₄(ATC) | | | | | | | | |
| | 0.15 mmol | | | | | | | | |
| MOF-20 | Zn(NO₃)₂·6H₂O | DMF | 90 | 92.13 | 90 | 8.13 | 16.444 | 12.807 | P2(1)/c |
| ZnNDC | 0.37 mmol | Chlorob | | | | | | | |
| | H₂NDC | enzene | | | | | | | |
| | 0.36 mmol | | | | | | | | |
| MOF-37 | Zn(NO₃)₂·6H₂O | DEF | 72.38 | 83.16 | 84.33 | 9.952 | 11.576 | 15.556 | P-1 |
| | 0.20 mmol | Chlorob | | | | | | | |
| | H₂NDC | enzene | | | | | | | |
| | 0.20 mmol | | | | | | | | |
| Zn(NDC) | Zn(NO₃)₂·6H₂O | DMSO | 68.08 | 75.33 | 88.31 | 8.631 | 10.207 | 13.114 | P-1 |
| (DMSO) | H₂NDC | | | | | | | | |
| Zn(NDC) | Zn(NO₃)₂·6H₂O | | 90 | 99.2 | 90 | 19.289 | 17.628 | 15.052 | C2/c |
| | H₂NDC | | | | | | | | |
| Zn(HPDC) | Zn(NO₃)₂·4H₂O | DMF | 107.9 | 105.06 | 94.4 | 8.326 | 12.085 | 13.767 | P-1 |
| | 0.23 mmol | H₂O | | | | | | | |
| | H₂(HPDC) | | | | | | | | |
| | 0.05 mmol | | | | | | | | |
| Co(HPDC) | Co(NO₃)₂·6H₂O | DMF | 90 | 97.69 | 90 | 29.677 | 9.63 | 7.981 | C2/c |
| | 0.21 mmol | H₂O/ | | | | | | | |
| | H₂ (HPDC) | ethanol | | | | | | | |
| | 0.06 mmol | | | | | | | | |
| Zn₃(PDC)2.5 | Zn(NO₃)₂·4H₂O | DMF/ | 79.34 | 80.8 | 85.83 | 8.564 | 14.046 | 26.428 | P-1 |
| | 0.17 mmol | CIBz | | | | | | | |
| | H₂(HPDC) | H₂0/ | | | | | | | |
| | 0.05 mmol | TEA | | | | | | | |
| Cd₂(TPDC)2 | Cd(NO₃)₂·4H₂O | Methano | 70.59 | 72.75 | 87.14 | 10.102 | 14.412 | 14.964 | P-1 |
| | 0.06 mmol | I/ CHP | | | | | | | |
| | H₂(HPDC) | H₂O | | | | | | | |
| | 0.06 mmol | | | | | | | | |
| Tb(PDC)1.5 | Tb(NO₃)₃·5H₂O | DMF | 109.8 | 103.61 | 100.14 | 9.829 | 12.11 | 14.628 | P-1 |
| | 0.21 mmol | H₂O/ | | | | | | | |
| | H₂(PDC) | ethanol | | | | | | | |
| | 0.034 mmol | | | | | | | | |
| ZnDBP | Zn(NO₃)₂·6H₂O | MeOH | 90 | 93.67 | 90 | 9.254 | 10.762 | 27.93 | P2/n |
| | 0.05 mmol | | | | | | | | |
| | dibenzylphosphate | | | | | | | | |
| | 0.10 mmol | | | | | | | | |
| Zn₃(BPDC) | ZnBr₂ | DMF | 90 | 102.76 | 90 | 11.49 | 14.79 | 19.18 | P21/n |
| | 0.021 mmol | | | | | | | | |
| | 4,4'BDC | | | | | | | | |
| | 0.005 mmol | | | | | | | | |
| CdBDC | Cd(NO₃)₂·4H₂O | DMF | 90 | 95.85 | 90 | 11.2 | 11.11 | 16.71 | P21/n |
| | 0.100 mmol | Na₂SiO₃ | | | | | | | |
| | H₂(BDC) | (aq) | | | | | | | |
| | 0.401 mmol | | | | | | | | |
| Cd-mBDC | Cd(NO₃)₂·4H₂O | DMF | 90 | 101.1 | 90 | 13.69 | 18.25 | 14.91 | C2/c |
| | 0.009 mmol | MeNH₂ | | | | | | | |
| | H₂(mBDC) | | | | | | | | |
| | 0.018 mmol | | | | | | | | |
| Zn₄OBNDC | Zn(NO₃)₂·6H₂O | DEF | 90 | 90 | 90 | 22.35 | 26.05 | 59.56 | Fmmm |
| | 0.041 mmol | MeNH₂ | | | | | | | |
| | BNDC | H₂O₂ | | | | | | | |
| Eu(TCA) | Eu(NO₃)₃·6H₂O | DMF | 90 | 90 | 90 | 23.325 | 23.325 | 23.325 | Pm-3n |
| | 0.14 mmol | Chlorob | | | | | | | |
| | TCA | enzene | | | | | | | |
| | 0.026 mmol | | | | | | | | |
| Tb(TCA) | Tb(NO₃)₃·6H₂O | DMF | 90 | 90 | 90 | 23.272 | 23.272 | 23.372 | Pm-3n |
| | 0.069 mmol | Chlorob | | | | | | | |
| | TCA | enzene | | | | | | | |
| | 0.026 mmol | | | | | | | | |
| Formate | Ce(NO₃)₃·6H₂O | H₂O | 90 | 90 | 120 | 10.668 | 10.667 | 4.107 | R-3m |
| | 0.138 mmol | Ethanol | | | | | | | |
| | Formaic acid | | | | | | | | |
| | 0.43 mmol | | | | | | | | |
| | FeCl₂·4H₂O | DMF | 90 | 90 | 120 | 8.2692 | 8.2692 | 63.566 | R-3c |
| | 5.03 mmol | | | | | | | | |
| | Formic acid | | | | | | | | |
| | 86.90 mmol | | | | | | | | |
| | FeCl₂·4H₂O | DEF | 90 | 90 | 90 | 9.9364 | 18.374 | 18.374 | Pbcn |
| | 5.03 mmol | | | | | | | | |
| | Formic acid | | | | | | | | |
| | 86.90 mmol | | | | | | | | |
| | FeCl₂·4H₂O | DEF | 90 | 90 | 90 | 8.335 | 8.335 | 13.34 | P-31c |
| | 5.03 mmol | | | | | | | | |
| | Formic acid | | | | | | | | |
| | 86.90 mmol | | | | | | | | |
| NO330 | FeCl₂·4H₂O | form- | 90 | 90 | 90 | 8.7749 | 11.655 | 8.3297 | Pnna |
| | 0.50 mmol | amide | | | | | | | |
| | Formic acid | | | | | | | | |
| | 8.69 mmol | | | | | | | | |
| N0332 | FeCl₂·4H₂O | DIP | 90 | 90 | 90 | 10.0313 | 18.808 | 18.355 | Pbcn |
| | 0.50 mmol | | | | | | | | |
| | Formic acid | | | | | | | | |
| | 8.69 mmol | | | | | | | | |
| NO333 | FeCl₂·4H₂O | DBF | 90 | 90 | 90 | 45.2754 | 23.861 | 12.441 | Cmcm |
| | 0.50 mmol | | | | | | | | |
| | Formic acid | | | | | | | | |
| | 8.69 mmol | | | | | | | | |
| NO335 | FeCl₂·4H₂O | CHF | 90 | 91.372 | 90 | 11.5964 | 10.187 | 14.945 | P21/n |
| | 0.50 mmol | | | | | | | | |
| | Formic acid | | | | | | | | |
| | 8.69 mmol | | | | | | | | |
| NO336 | FeCl₂·4H₂O | MFA | 90 | 90 | 90 | 11.7945 | 48.843 | 8.4136 | Pbcm |
| | 0.50 mmol | | | | | | | | |
| | Formic acid | | | | | | | | |
| | 8.69 mmol | | | | | | | | |
| NO13 | Mn(Ac)₂·4H₂O | Ethanol | 90 | 90 | 90 | 18.66 | 11.762 | 9.418 | Pbcn |
| | 0.46 mmol | | | | | | | | |
| | Bezoic acid | | | | | | | | |
| | 0.92 mmol | | | | | | | | |
| | Bipyridine | | | | | | | | |
| | 0.46 mmol | | | | | | | | |
| N029 | Mn(Ac)₂·4H₂O | DMF | 120 | 90 | 90 | 14.16 | 33.521 | 33.521 | P-1 |
| MOF-0 | 0.46 mmol | | | | | | | | |
| Like | H₃BTC | | | | | | | | |
| | 0.69 mmol | | | | | | | | |
| Mn(hfac)₂ | Mn(Ac)₂·4H₂O | Ether | 90 | 95.32 | 90 | 9.572 | 17.162 | 14.041 | C2/c |
| (O₂CC₆H₅) | 0.46 mmol | | | | | | | | |
| | Hfac | | | | | | | | |
| | 0.92 mmol | | | | | | | | |
| | Bipyridine | | | | | | | | |
| | 0.46 mmol | | | | | | | | |
| BPR43G2 | Zn(NO₃)₂·6H₂O | DMF | 90 | 91.37 | 90 | 17.96 | 6.38 | 7.19 | C2/c |
| | 0.0288 mmol | CH₃CN | | | | | | | |
| | H₂BDC | | | | | | | | |
| | 0.0072 mmol | | | | | | | | |
| BPR48A2 | Zn(NO₃)₂ 6H₂O | DMSO | 90 | 90 | 90 | 14.5 | 17.04 | 18.02 | Pbca |
| | 0.012 mmol | Toluene | | | | | | | |
| | H₂BDC | | | | | | | | |
| | 0.012 mmol | | | | | | | | |
| BPR49B1 | Zn(NO₃)₂ 6H₂O | DMSO | 90 | 91.172 | 90 | 33.181 | 9.824 | 17.884 | C2/c |
| | 0.024 mmol | Methanol | | | | | | | |
| | H₂BDC | | | | | | | | |
| | 0.048 mmol | | | | | | | | |
| BPR56E1 | Zn(NO₃)₂6H₂O | DMSO | 90 | 90.096 | 90 | 14.5873 | 14.153 | 17.183 | P2(1)/n |
| | 0.012 mmol | n- | | | | | | | |
| | H₂BDC | propanol | | | | | | | |
| | 0.024 mmol | | | | | | | | |
| BPR68D10 | Zn(NO₃)₂ 6H₂O | DMSO | 90 | 95.316 | 90 | 10.0627 | 10.17 | 16.413 | P2(1)/c |
| | 0.0016 mmol | Benzene | | | | | | | |
| | H₃BTC | | | | | | | | |
| | 0.0064 mmol | | | | | | | | |
| BPR69B1 | Cd(NO₃)₂ 4H₂O | DMSO | 90 | 98.76 | 90 | 14.16 | 15.72 | 17.66 | Cc |
| | 0.0212 mmol | | | | | | | | |
| | H₂BDC | | | | | | | | |
| | 0.0428 mmol | | | | | | | | |
| BPR73E4 | Cd(NO₃)₂ 4H₂O | DMSO | 90 | 92.324 | 90 | 8.7231 | 7.0568 | 18.438 | P2(1)/n |
| | 0.006 mmol | Toluene | | | | | | | |
| | H₂BDC | | | | | | | | |
| | 0.003 mmol | | | | | | | | |
| BPR76D5 | Zn(NO₃)₂ 6H₂O | DMSO | 90 | 104.17 | 90 | 14.4191 | 6.2599 | 7.0611 | Pc |
| | 0.0009 mmol | | | | | | | | |
| | H₂BzPDC | | | | | | | | |
| | 0.0036 mmol | | | | | | | | |
| BPR80B5 | Cd(NO₃)₂·4H₂O | DMF | 90 | 115.11 | 90 | 28.049 | 9.184 | 17.837 | C2/c |
| | 0.018 mmol | | | | | | | | |
| | H₂BDC | | | | | | | | |
| | 0.036 mmol | | | | | | | | |
| BPR80H5 | Cd(NO₃)₂ 4H₂O | DMF | 90 | 119.06 | 90 | 11.4746 | 6.2151 | 17.268 | P2/c |
| | 0.027 mmol | | | | | | | | |
| | H₂BDC | | | | | | | | |
| | 0.027 mmol | | | | | | | | |
| BPR82C6 | Cd(NO₃)₂ 4H₂O | DMF | 90 | 90 | 90 | 9.7721 | 21.142 | 27.77 | Fdd2 |
| | 0.0068 mmol | | | | | | | | |
| | H₂BDC | | | | | | | | |
| | 0.202 mmol | | | | | | | | |
| BPR86C3 | Co(NO₃)₂ 6H₂O | DMF | 90 | 90 | 90 | 18.3449 | 10.031 | 17.983 | Pca2(1) |
| | 0.0025 mmol | | | | | | | | |
| | H₂BDC | | | | | | | | |
| | 0.075 mmol | | | | | | | | |
| BPR86H6 | Cd(NO₃)₂·6H₂O | DMF | 80.98 | 89.69 | 83.412 | 9.8752 | 10.263 | 15.362 | P-1 |
| | 0.010 mmol | | | | | | | | |
| | H₂BDC | | | | | | | | |
| | 0.010 mmol | | | | | | | | |
| | Co(NO₃)₂ 6H₂O | NMP | 106.3 | 107.63 | 107.2 | 7.5308 | 10.942 | 11.025 | P1 |
| BPR95A2 | Zn(NO₃)₂ 6H₂O | NMP | 90 | 102.9 | 90 | 7.4502 | 13.767 | 12.713 | P2(1)/c |
| | 0.012 mmol | | | | | | | | |
| | H₂BDC | | | | | | | | |
| | 0.012 mmol | | | | | | | | |
| CuC₆F₄O₄ | Cu(NO₃)₂·2.5H₂O | DMF | 90 | 98.834 | 90 | 10.9675 | 24.43 | 22.553 | P2(1)/n |
| | 0.370 mmol | Chloro- | | | | | | | |
| | H₂BDC(OH)₂ | Benzene | | | | | | | |
| | 0.37 mmol | | | | | | | | |
| Fe Formic | FeCl₂·4H₂O | DMF | 90 | 91.543 | 90 | 11.495 | 9.963 | 14.48 | P2(1)/n |
| | 0.370 mmol | | | | | | | | |
| | Formic acid | | | | | | | | |
| | 0.37 mmol | | | | | | | | |
| Mg Formic | Mg(NO₃)₂·6H₂O | DMF | 90 | 91.359 | 90 | 11.383 | 9.932 | 14.656 | P2(1)/n |
| | 0.370 mmol | | | | | | | | |
| | Formic acid | | | | | | | | |
| | 0.37 mmol | | | | | | | | |
| MgC₆H₄O₆ | Mg(NO₃)₂·6H₂O | DMF | 90 | 96.624 | 90 | 17.245 | 9.943 | 9.273 | C2/c |
| | 0.370 mmol | | | | | | | | |
| | H₂BDC(OH)₂ | | | | | | | | |
| | 0.37 mmol | | | | | | | | |
| Zn C₂H₄BDC | ZnCl₂ | DMF | 90 | 94.714 | 90 | 7.3386 | 16.834 | 12.52 | P2(1)/n |
| MOF-38 | 0.44 mmol | | | | | | | | |
| | CBBDC | | | | | | | | |
| | 0.261 mmol | | | | | | | | |
| MOF-49 | ZnCl₂ | DMF | 90 | 93.459 | 90 | 13.509 | 11.984 | 27.039 | P2/c |
| | 0.44 mmol m-BDC 0.261 mmol | CH3CN | | | | | | | |
| MOF-26 | Cu(NO₃)₂·5H₂O | DMF | 90 | 95.607 | 90 | 20.8797 | 16.017 | 26.176 | P2(1)/n |
| | 0.084 mmol | | | | | | | | |
| | DCPE | | | | | | | | |
| | 0.085 mmol | | | | | | | | |
| MOF-112 | Cu(NO₃)₂·2.5H₂O | DMF | 90 | 107.49 | 90 | 29.3241 | 21.297 | 18.069 | C2/c |
| | 0.084 mmol | Ethanol | | | | | | | |
| | *o*-Br-*m*-BDC | | | | | | | | |
| | 0.085 mmol | | | | | | | | |
| MOF-109 | Cu(NO₃)₂·2.5H₂O | DMF | 90 | 111.98 | 90 | 23.8801 | 16.834 | 18.389 | P2(1)/c |
| | 0.084 mmol | | | | | | | | |
| | KDB | | | | | | | | |
| | 0.085 mmol | | | | | | | | |
| MOF-111 | Cu(NO₃)₂·2.5H₂O | DMF | 90 | 102.16 | 90 | 10.6767 | 18.781 | 21.052 | C2/c |
| | 0.084 mmol | Ethanol | | | | | | | |
| | o-BrBDC | | | | | | | | |
| | 0.085 mmol | | | | | | | | |
| MOF-110 | Cu(NO₃)₂·2.5H₂O | DMF | 90 | 90 | 120 | 20.0652 | 20.065 | 20.747 | R-3/m |
| | 0.084 mmol | | | | | | | | |
| | thiophene | | | | | | | | |
| | dicarboxylic | | | | | | | | |
| | 0.085 mmol | | | | | | | | |
| MOF-107 | Cu(NO₃)₂·2.5H₂O | DEF | 104.8 | 97.075 | 95.206 | 11.032 | 18.067 | 18.452 | P-1 |
| | 0.084 mmol | | | | | | | | |
| | thiophene | | | | | | | | |
| | dicarboxylic | | | | | | | | |
| | 0.085 mmol | | | | | | | | |
| MOF-108 | Cu(NO₃)₂·2.5H₂O | DBF/ | 90 | 113.63 | 90 | 15.4747 | 14.514 | 14.032 | C2/c |
| | 0.084 mmol | methanol | | | | | | | |
| | thiophene | | | | | | | | |
| | dicarboxylic | | | | | | | | |
| | 0.085 mmol | | | | | | | | |
| MOF-102 | Cu(NO₃)₂·2.5H₂O | DMF | 91.63 | 106.24 | 112.01 | 9.3845 | 10.794 | 10.831 | P-1 |
| | 0.084 mmol | | | | | | | | |
| | H₂(BDCCl₂) | | | | | | | | |
| | 0.085 mmol | | | | | | | | |
| Clbdc1 | Cu(NO₃)₂·2.5H₂O | DEF | 90 | 105.56 | 90 | 14.911 | 15.622 | 18.413 | P-1 |
| | 0.084 mmol | | | | | | | | |
| | H₂(BDCCl₂) | | | | | | | | |
| | 0.085 mmol | | | | | | | | |
| Cu(NMOP) | Cu(NO₃)₂·2.5H₂O | DMF | 90 | 102.37 | 90 | 14.9238 | 18.727 | 15.529 | P2(1)/m |
| | 0.084 mmol | | | | | | | | |
| | NBDC | | | | | | | | |
| | 0.085 mmol | | | | | | | | |
| Tb(BTC) | Tb(NO₃)₃·5H₂O | DMF | 90 | 106.02 | 90 | 18.6986 | 11.368 | 19.721 | |
| | 0.033 mmol | | | | | | | | |
| | H₃BTC | | | | | | | | |
| | 0.033 mmol | | | | | | | | |
| Zn₃(BTC)₂ | ZnCl₂ | DMF | 90 | 90 | 90 | 26.572 | 26.572 | 26.572 | Fm-3m |
| Honk | 0.033 mmol | Ethanol | | | | | | | |
| | H₃BTC | | | | | | | | |
| | 0.033 mmol | | | | | | | | |
| Zn₄O(NDC) | Zn(NO₃)₂·4H₂O | DMF | 90 | 90 | 90 | 41.5594 | 18.818 | 17.574 | aba2 |
| | 0.066 mmol | ethanol | | | | | | | |
| | 14NDC | | | | | | | | |
| | 0.066 mmol | | | | | | | | |
| CdTDC | Cd(NO₃)₂·4H₂O | DMF | 90 | 90 | 90 | 12.173 | 10.485 | 7.33 | Pmma |
| | 0.014 mmol | H₂O | | | | | | | |
| | thiophene | | | | | | | | |
| | 0.040 mmol | | | | | | | | |
| | DABCO | | | | | | | | |
| | 0.020 mmol | | | | | | | | |
| IRMOF-2 | Zn(NO₃)₂·4H₂O | DEF | 90 | 90 | 90 | 25.772 | 25.772 | 25.772 | Fm-3m |
| | 0.160 mmol | | | | | | | | |
| | o-Br-BDC | | | | | | | | |
| | 0.60 mmol | | | | | | | | |
| IRMOF-3 | Zn(NO₃)₂·4H₂O | DEF | 90 | 90 | 90 | 25.747 | 25.747 | 25.747 | Fm-3m |
| | 0.20 mmol | Ethanol | | | | | | | |
| | H₂N-BDC | | | | | | | | |
| | 0.60 mmol | | | | | | | | |
| IRMOF-4 | Zn(NO₃)₂·4H₂O | DEF | 90 | 90 | 90 | 25.849 | 25.849 | 25.849 | Fm-3m |
| | 0.11 mmol | | | | | | | | |
| | [C₃H₇O]₂-BDC | | | | | | | | |
| | 0.48 mmol | | | | | | | | |
| IRMOF-5 | Zn(NO₃)₂·4H₂O | DEF | 90 | 90 | 90 | 12.882 | 12.882 | 12.882 | Pm-3m |
| | 0.13 mmol | | | | | | | | |
| | [C₅H₁₁O]₂-BDC | | | | | | | | |
| | 0.50 mmol | | | | | | | | |
| IRMOF-6 | Zn(NO₃)₂·4H₂O | DEF | 90 | 90 | 90 | 25.842 | 25.842 | 25.842 | Fm-3m |
| | 0.20 mmol | | | | | | | | |
| | [C₂H₄]-BDC | | | | | | | | |
| | 0.60 mmol | | | | | | | | |
| IRMOF-7 | Zn(NO₃)₂·4H₂O | DEF | 90 | 90 | 90 | 12.914 | 12.914 | 12.914 | Pm-3m |
| | 0.07 mmol | | | | | | | | |
| | 1,4NDC | | | | | | | | |
| | 0.20 mmol | | | | | | | | |
| IRMOF-8 | Zn(NO₃)₂·4H₂O | DEF | 90 | 90 | 90 | 30.092 | 30.092 | 30.092 | Fm-3m |
| | 0.55 mmol | | | | | | | | |
| | 2,6NDC | | | | | | | | |
| | 0.42 mmol | | | | | | | | |
| IRMOF-9 | Zn(NO₃)₂-4H₂O | DEF | 90 | 90 | 90 | 17.147 | 23.322 | 25.255 | Pnnm |
| | 0.05 mmol | | | | | | | | |
| | BPDC | | | | | | | | |
| | 0.42 mmol | | | | | | | | |
| ERMOF-10 | Zn(NO₃)₂·4H₂O | DEF | 90 | 90 | 90 | 34.281 | 34.281 | 34.281 | Fm-3m |
| | 0.02 mmol | | | | | | | | |
| | BPDC | | | | | | | | |
| | 0.012 mmol | | | | | | | | |
| IRMOF-11 | Zn(NO₃)₂·4H₂O | DEF | 90 | 90 | 90 | 24.822 | 24.822 | 56.734 | R-3m |
| | 0.05 mmol | | | | | | | | |
| | HPDC | | | | | | | | |
| | 0.20 mmol | | | | | | | | |
| IRMOF-12 | Zn(NO₃)₂·4H₂O | DEF | 90 | 90 | 90 | 34.281 | 34.281 | 34.281 | Fm-3m |
| | 0.017 mmol | | | | | | | | |
| | HPDC | | | | | | | | |
| | 0.12 mmol | | | | | | | | |
| IRMOF-13 | Zn(NO₃)₂·4H₂O | DEF | 90 | 90 | 90 | 24.822 | 24.822 | 56.734 | R-3m |
| | 0.048 mmol | | | | | | | | |
| | PDC | | | | | | | | |
| | 0.31 mmol | | | | | | | | |
| IRMOF-14 | Zn(NO₃)₂·4H₂O | DEF | 90 | 90 | 90 | 34.381 | 34.381 | 34.381 | Fm-3m |
| | 0.17 mmol | | | | | | | | |
| | PDC | | | | | | | | |
| | 0.12 mmol | | | | | | | | |
| IRMOF-15 | Zn(NO₃)₂·4H₂O | DEF | 90 | 90 | 90 | 21.459 | 21.459 | 21.459 | Im-3m |
| | 0.063 mmol | | | | | | | | |
| | TPDC | | | | | | | | |
| | 0.025 mmol | | | | | | | | |
| IRMOF-16 | Zn(NO₃)₂·4H₂O | DEF | 90 | 90 | 90 | 21.49 | 21.49 | 21.49 | Pm-3m |
| | 0.0126 mmol | NMP | | | | | | | |
| | TPDC | | | | | | | | |
| | 0.05 mmol | | | | | | | | |
| | FeBr₂ 0.927 mmol | DMF | | | | | | | |
| | BDC 0.927 mmol | 1 Propanol | | | | | | | |
| | FeCl₃·6H₂O | DMF | | | | | | | |
| | BDC 1.23 mmol | Ethanol | | | | | | | |
| | Mg(NO₃)₂·6H₂O | DMF | | | | | | | |
| | DHBC 0.185 mmol | | | | | | | | |
| | Zn(NO₃)₂·4H₂O | DMF | | | | | | | |
| | 0.20 mmol | i-Propanol | 90 | 90 | 120 | 25,9 | 25,9 | 6,8 | R-3 |
| | DHBC 0.10 mmol | | | | | | | | |
| | Mn(ClO₄)₂·6H₂O | DMF | | | | | | | |
| | DHBC 0.065 mmol | i-Propanol | | | | | | | |
| | Tb(NO₃)₃·5H₂O | DMF | | | | | | | |
| | DHBC 0.050 mmol | i-Propanol | | | | | | | |

| | | | | | | | | | |
|---|---|---|---|---|---|---|---|---|---|
| ADC Acetylene dicarboxylic acid NDC Naphtalene dicarboxylic acid BDC Benzene dicarboxylic acid ATC Adamantane tetracarboxylic acid BTC Benzene tricarboxylic acid BTB Benzene tribenzoate MTB Methane tetrabenzoate ATB Adamantane tetrabenzoate ADB Adamantane dibenzoate BPDC 4,4-Biphenyldicarboxylic acid DHBC 2,5-Dihydroxyterephthalic acid | | | | | | | | | |

Examples for the synthesis of these materials as such can, for example, be found in: J. Am. Chem. Soc. 123 (2001) pages 8241 seq. or in Acc. Chem. Res. 31 (1998) pages 474 seq.

The separation of the framework materials from the mother liquor of the crystallization can be achieved by procedures known in the art such as solid-liquid separations, centrifugation, extraction, filtration, membrane filtration, cross-flow filtration, flocculation using flocculation adjuvants (non-ionic, cationic and anionic adjuvants) or by the addition of pH shifting additives such as salts, acids or bases, by flotation, as well as by evaporation of the mother liquor at elevated temperature and/or in vacuo and concentrating of the solid. The material obtained in this step is typically a fine powder and cannot be used for most practical applications, e.g., in catalysis, where shaped bodies are required.

The separated framework materials may be compounded, melted, extruded, co-extruded, pressed, spinned, foamed and granulated according to processes known within the processing of plastics, respectively.

One advantage of the process according to the present invention is that the polyoxyalkylene alcohols obtainable have a low degree of alkoxylation. The alcohols comprise 1 to 5 alkoxy units, preferably 1 to 3 alkoxy units, more preferably 1 or 2 alkoxy units, in particular 1 alkoxy unit.

The polyoxyalkylene alcohols which are obtainable according to the process of the present invention lend themselves for a number of applications. Non-limiting examples include polyurethane-foams, lubricating liquids, hydraulic fluid, carrier liquid, tenside and flotation oil.

The invention is now illustrated by way of the following examples.

### Examples

### Example 1 (Preparation of MOF-5)

| **Starting Material** | **Molar Amount** | **Calculated** | **Experimental** |
|---|---|---|---|
| terephthalic acid | 12.3 mmol | 2.04 g | 2.34 g |
| Zinc nitrate-tetra hydrate | 36.98 mmol | 9.67 g | 9.66 g |
| Diethylformamide (Merck) | 2568.8 mmol | 282.2 g | 282.2 g |

The above-mentioned amounts of the starting materials were dissolved in a beaker in the order diethylformamide, terephthalic acid and zinc nitride. The resulting solution was transferred into two autoclaves (250 ml) with teflon covered inner walls.

The crystallization occurred at 105°C over 68 hours. Subsequently, the orange solvent, together with the red crystals, was transferred into a beaker, and the suspension is filtered unter an N2 atmosphrere. The suspension was washed with 3 ml of chloroform before being activated *in vacuo*. There were obtained 2.3 g of product.

### Example 2:

2,5-Dihydroxyterephthalic acid (19 mg, 0,10 mmol) and Zn(NO₃)₂•4H₂O (53 mg, 0.20 mmol) were dissolved in a mixed solution of DMF (2.0 mL), PrOH(0.10 mL) and water (0.10 mL), which was placed in a pyrex tube (10 mm x 70 mm). The tube was frozen and evacuated, and flame sealed under vacuum. The tube was heated to 105°C at 2°C/min, held for 20 hours, then cooled to room temperature at 2°C/min. Yellow needle crystals were collected and washed with DMF (3 x 5 mL). Yield: 26 mg, 81 % based on the 2,5-dihydroxyterephthalic acid.

### Example 3 (Alkoxylation of i-Tridecanol N with Propylene Oxide)

i-Tridecanol N (4,8 g corresponding to 0.024 mole) and 0.8 g of the catalyst prepared according to Example 1 were given into an autoclave. Subsequently, the autoclave was filled with 12 g propylene oxide (0.207 mole). The reaction was carried out at 135°C, and in total 9.4 mole propylene oxide/mole starting alcohol were reacted to obtain 18.7 g of product.

### Example 4 (Alkoxylation of 2-Propylheptanol with Ethylene Oxide)

2-Propylheptanol (12.67 g corresponding to 0.08 mole) and 0.49 g of the catalyst prepared according to Example 2 were given into an autoclave. Subsequently, the autoclave was filled with 7.05 g ethylene oxide (0.16 mole). The reaction was carried out at 135°C over 10 h, before the autocalve was cooled to 50°C, at which temperature the reaction mixture was stirred for another 3 h. In total 3.74 mole ethylene oxide/mole starting alcohol were reacted, to obtain 27.98 g of product.

## Claims

1. A process for the alkoxylation of a monool with at least one alkoxylating agent to a polyoxyalkylene alcohol wherein the polyoxyalkylene alcohol comprises one to five alkoxy units and wherein a catalyst is employed which comprises a metallo-organic framework material of metal ions and at least bidentate coordinately bound organic ligands.

2. The process according to claim 1, wherein the metal ion is selected among ions of elements of groups Ia, IIa, IIIa, IVa to VIIIa and Ib to VIb of the periodic table of the elements.

3. The process according to claim 1 or 2, wherein the bidentate organic ligand is selected among substituted and unsubstituted mono- and polynuclear aromatic polycarboxylic acids and substituted or unsubstituted aromatic mono- and polynuclear polycarboxylic acids which comprise at least one hetero atom.

4. The process according to claim 3, wherein the ligand is terephthalic acid or a derivative thereof.

5. The process according to any of claims 1 to 4, wherein the metallo-organic framework material exhibits a specific surface area, as determined via adsorption, of > 20 m²/g.

6. The process according to any of claims 1 to 5, wherein the alkoxylation agent is selected among mono- and multifunctional epoxides having 2 to 30 carbon atoms mixtures of two or more thereof.

7. The process according to claim 6, wherein the epoxide is selected from ethylene oxide, propylene oxide, butylenes oxides and mixtures thereof.

## Patentansprüche

1. Verfahren zur Alkoxylierung eines Monools mit mindestens einem Alkylierungsmittel zu einem Polyoxyalkylenalkohol, bei dem der Polyoxyalkylenalkohol eins bis fünf Alkoxyeinheiten umfaßt und ein Katalysator eingesetzt wird, der ein metallorganisches Gerüstmaterial aus Metallionen und mindestens zweizähnigen, koordinativ gebundenen organischen Liganden umfaßt.

2. Verfahren nach Anspruch 1, bei dem man das Metallion unter Ionen von Elementen der Gruppen Ia, IIa, lila, IVa bis VIIIa und. Ib bis VIb des Periodensystems der Elemente auswählt.

3. Verfahren nach Anspruch 1 oder 2, bei dem man den zweizähnigen organischen Liganden unter substituierten und unsubstituierten ein- und mehrkernigen aromatischen Polycarbonsäuren und substituierten oder unsubstituierten aromatischen ein- oder mehrkernigen Polycarbonsäuren mit mindestens einem Heteroatom auswählt.

4. Verfahren nach Anspruch 3, bei dem es sich bei dem Liganden um Terephthalsäure oder ein Derivat davon handelt.

5. Verfahren nach einem der Ansprüche 1 bis 4, bei dem das metallorganische Gerüstmaterial eine durch Adsorption bestimmte spezifische Oberfläche von > 20 m²/g aufweist.

6. Verfahren nach einem der Ansprüche 1 bis 5, bei dem man das Alkoxylierungsmittel unter mono- und multifunktionellen Epoxiden mit 2 bis 30 Kohlenstoffatomen und Mischungen von zwei oder mehr dieser Verbindungen auswählt.

7. Verfahren nach Anspruch 6, bei dem man das Epoxid unter Ethylenoxid, Propylenoxid, Butylenoxiden und Mischungen davon auswählt.

## Revendications

1. Procédé d'alcoxylation d'un monool avec au moins un agent d'alcoxylation pour former un polyoxyalkylène alcool, dans lequel le polyoxyalkylène alcool comprend une à cinq unités alcoxy et dans lequel un catalyseur, qui comprend un matériau de cadre métallo-organique d'ions métalliques et des ligands organiques au moins bidentates coordinativement reliés, est employé.

2. Procédé selon la revendication 1, dans lequel l'ion métallique est choisi parmi les ions des éléments des groupes Ia, IIa, IIIa, IVa à VIIIa et Ib à VIb du tableau périodique des éléments.

3. Procédé selon la revendication 1 ou 2, dans lequel le ligand organique bidentate est choisi parmi les acides polycarboxyliques aromatiques mono- et polynucléaires substitués et non substitués et les acides polycarboxyliques mono- et polynucléaires aromatiques substitués et non substitués qui comprennent au moins un hétéroatome.

4. Procédé selon la revendication 3, dans lequel le ligand est l'acide téréphtalique ou un de ses dérivés.

5. Procédé selon l'une quelconque des revendications 1 à 4, dans lequel le matériau de cadre métallo-organique présente une aire de surface spécifique, telle que déterminée par adsorption, > 20 m²/g_{.}

6. Procédé selon l'une quelconque des revendications 1 à 5, dans lequel l'agent d'alcoxylation est choisi parmi les époxydes mono- et multifonctionnels contenant 2 à 30 atomes de carbone et les mélanges de deux ou plus d'entre eux.

7. Procédé selon la revendication 6, dans lequel l'époxyde est choisi parmi l'oxyde d'éthylène, l'oxyde de propylène, les oxydes de butylènes et leurs mélanges.
